# EUROPÄISCHE PATENTSCHRIFT

(11) **EP 2 415 762 B1**
(45) Veröffentlichungstag und Bekanntmachung des Hinweises auf die Patenterteilung: **23.04.2014**
(21) Anmeldenummer: 11175821.5
(22) Anmeldetag: 28.07.2011
(51) Int. Cl.: C07D 213/75

(54) **Verfahren zur Herstellung von Aminoarylalkylverbindungen**
Method for manufacturing amino-aryl alkyl compounds
Procédé de fabrication de liaisons aminoarylalkyles

(30) Priorität: 06.08.2010 DE 102010033690
(43) Veröffentlichungstag der Anmeldung: 08.02.2012
(73) Patentinhaber: Saltigo GmbH, 51369 Leverkusen (DE)
(72) Erfinder: Sander, Michael, 51503 Rösrath (DE); Stirner, Wolfgang, 51467 Bergisch Gladbach (DE); Laschinski, Frank, 51377 Leverkusen (DE); Konrad, Michael, 51379 Leverkusen (DE); Von Dem Bruch, Karsten, 51371 Leverkusen (DE)
(74) Vertreter: Pettrich, Klaus-Günter

(56) Entgegenhaltungen:
- WO-A2-2009/005638
- WO-A2-2009/017822

## Beschreibung

Die Erfindung betrifft ein Verfahren zur Herstellung von Aminoarylalkylverbindungen, insbesondere des 5-Amino-2-isopropylpyridin.

Aminoarylalkylverbindungen, insbesondere das 5-Amino-2-isopropylpyridin, stellen wertvolle Zwischenprodukte zur Synthese von Arzneimitteln dar. In EP 1852431 A wird beschrieben das (1S)-(-)-N-[(1-Ethyl-1H-pyrazol-4-yl)methyl]-5-hydroxy-N-(6-isopropylpyridin-3-yl)-1,2,3,4-tetrahydronaphtalene-1-carboxamide als prophylaktisches oder/und therapeutisches, entzündungshemmendes Arzneimittel verwendet werden kann und aus 5-Amino-2-isopropylpyridin hergestellt wird. 5-Amino-2-isopropylpyridin lässt sich durch Schutzgruppenabspaltung aus einer N-geschützten Aminoarylalkylverbindung, nämlich aus dem 5-(N-(Boc)-amino)-2-isopropylpyridine, herstellen.

Aus EP 1852431 A ist zudem bekannt, dass 5-Amino-2-isopropylpyridin ausgehend von 2-Hydroxy-6-isopropylnicotinnitrile hergestellt werden kann. JP 2008-222593 A beschreibt ein Verfahren zur Herstellung von 5-Amino-2-isopropylpyridin ausgehend von 2-Isopropylpyridin-5-carboxyamide durch Hofmann-Abbau unter Verwendung von Natriumhypochlorit.

Den bisherigen Verfahren zur Herstellung von Aminoarylalkylverbindungen, insbesondere des 5-Amino-2-isopropylpyridins, ist gemeinsam, dass sie in technischen Prozessen nicht effizient eingesetzt werden können und zudem in Hinblick auf die Anzahl der Verfahrensschritte zu niedrige Ausbeuten liefern.

Es bestand daher weiterhin ein Bedürfnis nach einem Verfahren zur Herstellung von Aminoarylalkylverbindungen mit dem die Nachteile des Standes der Technik überwunden werden können und Aminoarylalkylverbindungen in guten Ausbeuten und effizient in technischen Prozessen hergestellt werden können.

Überraschend wurde gefunden, dass Aminoarylalkylverbindungen ausgehend von N,N-geschützten Aminoarylalkylhalogenverbindungen und Eisen katalysierte Kopplung mit Grignardverbindungen zu N-geschützten Aminoarylalkylverbindung in guten Ausbeuten umgesetzt werden können. Diese N-geschützten Aminoarylalkylverbindung können dann durch Schutzgruppenabspaltung in die Aminoarylalkyverbindung überführt werden.

Gegenstand der Erfindung ist daher ein Verfahren zur Herstellung der Verbindungen der Formel (1)

R¹-NH-ARYL-R² (1)

wobei R¹ = -COOR³ oder -SO₂-R⁴ wobei R³ und R⁴ ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, Cl, Br oder F oder R¹ = -SO₂-NH-(C₁-C₁₀-Alkyl), -SO₂-NH-(C₇-C₁₅-Arylalkyl), - SO₂NH-(C₆-C₂₄-Aryl) oder -SO₂(NR⁵R⁶) wobei R⁵ und R⁶ für C₁-C₁₀-Alkyl stehen oder NR⁵R⁶ bilden zusammen einen 5- bis 7-gliedrigen Ring und
ARYL für einen unsubstituierten oder substituierten carbocyclischen C₆-C₂₄-Aryl- oder unsubstituierten oder substituierten heteroaromatischen C₃-C1₆₋Heteroarylrest steht und
R² = C₁-C₁₀-Alkyl, C₂-C₁₀-Alkenyl, C₆-C₂₄-Aryl, C₇-C₁₅-Arylalkyl, C₃-C₁₆-Heteroaryl oder 3- bis 7- gliedriger gesättigter oder teilweise ungesättigter Heterozyklus, welche gegebenenfalls weiter substituiert sein können durch Reste ausgewählt aus der Gruppe: C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₂-(C₇-C₁₅-Arylalkyl), -SO₃-(C₇-C₁₅-Arylalkyl), -SO₃(C₆-C₂₄-Aryl), -SO₃(C₁-C₁₀-Alkyl), -COO-(C₆₋C₂₄-Aryl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder - SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7-gliedrigen Ring, bei dem Verbindungen der Formel (2) 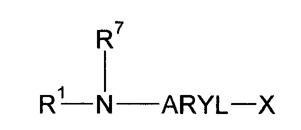 wobei R¹ und R⁷ gleich oder verschieden sein können und R¹ und ARYL die o.g. Bedeutungen besitzen und R⁷= -COOR³ oder -SO₂-R⁴ wobei R³ und R⁴ und ausgewählt sind aus der Gruppe: C₁-C₁₀-Alkyl, C₂-C₁₀-Alkenyl, C₁-C₁₀-Perhalogenalkyl, C₇-C₁₅-Arylalkyl, C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, Cl, Br oder F oder R¹ = -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁵R⁶) wobei R⁵ und R⁶ für C₁-C₁₀-Alkyl stehen oder NR⁵R⁶ bilden zusammen einen 5- bis 7-gliedrigen Ring und
X = Cl, Br, I oder-OSO₂-R⁸ mit R⁸ = C₁-C₁₀-Alkyl, C₁-C₁₀-Perhalogenalkyl, C₇-C₁₅-Arylalkyl, C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F, in Gegenwart von mindestens einer Eisenquelle mit Verbindungen der Formel (3) 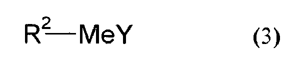 in denen R² die vorgenannte Bedeutung besitzt und Y = anionischer Ligand und Me ein Metall ausgewählt aus der Gruppe Mg, Ca, Mn, Zn ist, zu Verbindungen der Formel (1) umgesetzt werden.

ARYL stellt vorzugsweise einen unsubstituierten C₆-C₂₄-Aryl oder unsubstituierten C₃-C₁₆-Heteroaryl oder einen durch ein bis drei gleiche oder verschiedene Substituenten substiuierten C₆-C₂₄-Aryl oder substituierten C₃-C₁₆-Heteroaryl dar, wobei die Substituenten ausgewählt sind aus der Gruppe: C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -COO-(C₆-C₂₄-Aryl), -OCOO-(C₁-C₁₀-Alkyl), -OCOO-(C₇-C₁₅-Arylalkyl), -OCOO-(C₆-C₂₄-Aryl), -SO₃-(C₇-C₁₅-Arylalkyl), - SO₃(C₆-C₂₄-Aryl), -SO₃(C₁-C₁₀-Alkyl), -SO₂-(C₇-C₁₅-Arylalkyl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring.

In einer Ausführungsform der Erfindung steht ARYL bevorzugt für einen Phenylrest, der gegebenenfalls durch ein bis drei gleiche oder verschiedene Reste substituiert sein kann ausgewählt aus der Gruppe : C₁-C₁₀-Alkyl, C₂₋C₁₀₋Alkenyl, C₁₋C₁₀₋Alkoxy, C₁-C₁₀₋Perhalogenalkyl, C₂-C₁₀₋Alkynyl, C₆-C₂₄-Aryl, C₃-C₁₆-Heteroaryl, -COO-(C₁-C₁₀-Alkyl), -COO-(C₆-C₂₄-Aryl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₃-(C₇-C₁₅-Arylalkyl), -SO₃(C₆-C₂₄-Aryl), -SO₃(C₁-C₁₀-Alkyl), -SO₂-(C₇-C₁₅-Arylalkyl), -SO₂(C₁-C₁₀-Alkyl), - SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring.

In einer anderen Ausführungsform der Erfindung steht ARYL bevorzugt für einen Pyridyl, Pyrimidyl, Pyridazinyl oder Pyrazinylrest, der gegebenenfalls durch ein bis drei gleiche oder verschiedene Reste substituiert sein kann ausgewählt aus der Gruppe : C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₆-C₂₄-Aryl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₃-(C₇-C₁₅-Arylalkyl), -SO₃(C₆-C₂₄-Aryl), -SO₃(C₁-C₁₀-Alkyl), -SO₂-(C₇-C₁₅-Arylalkyl), -SO₂(C1-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder - SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7-gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring.

In einer anderen Ausführungsform der Erfindung steht ARYL besonders bevorzugt für einen Pyridylrest, der gegebenenfalls durch ein bis drei gleiche oder verschiedene Reste substituiert sein kann ausgewählt aus der Gruppe C₁-C₁₀-Alkyl, C₂-C₁₀-Alkenyl, C1-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₆-C₂₄-Aryl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₃-(C₇-C₁₅-Arylalkyl), -SO₃(C₆-C₂₄-Aryl), -SO₃(C₁-C₁₀-Alkyl), -SO₂-(C₇-C₁₅-Arylalkyl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring.

In einer besonders bevorzugten Ausführungsform der Erfindung steht ARYL für 2-, 3- oder 4-Pyridinyl.

R¹ oder/und R⁷ stehen bevorzugt für -COOR³ oder -SO₂₋R⁴ wobei R³ und R⁴ gleich oder verschieden sein können und ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀Alkyl oder C₆-C₂₄-Aryl die einfach oder mehrfach, aber nicht vollständig substituiert sind durch C₁-C₁₀-Alkoxy, Cl, Br oder F. Bevorzugt steht R³ für C₁-C₁₀-Alkyl, C₆-C₂₄-Aryl oder C₂-C₁₀-Alkenyl. Besonders bevorzugt steht R³ für tert.-Butyl. Bevorzugt steht R⁴ für ein C₁-C₁₀-Alkyl oder C₆-C₂₄-Aryl die einfach oder mehrfach, aber nicht vollständig substituiert sind durch C₁-C₁₀-Alkoxy, Cl, Br oder F oder R⁴steht für C₁-C₁₀-Perhalogenalkyl. Besonders bevorzugt stehen R¹ oder/und R⁷für -(CO)-O-(tert.Butyl), - (CO)-O-(Allyl) -(CO)-O-(Methyl), -(CO)-O-(Ethyl), -(CO)-O-(s-Propyl), -(CO)-O-(n-Propyl), - (CO)-O-(n-Butyl), -(CO)-O-(s-Butyl), -(CO)-O-(i-Butyl), -(CO)-O-(neo-Pentyl), -(CO)-O-(Nonafluorbutyl), -SO₂-(Benzyl), -SO₂-(Dimethylbenzyl), -SO₂-(Trimethylbenzyl), -SO₂-(Phenyl), - SO₂-(o-Tolyl), -SO₂-(p-Tolyl), -SO₂-(m-Tolyl), -SO₂-(Difluorbenzyl) oder -SO₂-(Trifluorbenzyl). Ganz besonders bevorzugt stehen R¹ oder/und R⁷ für -COO-(tert.Butyl).

Bevorzugt sind R¹ und R⁷ gleich.

Bevorzugt steht R² für C₁-C₁₀-Alkyl, C₂-C₁₀-Alkenyl, C₃-C₁₆-Heteroaryl oder C₆-C₂₄-Aryl, welche gegebenenfalls weiter substituiert sein können durch Reste ausgewählt aus der Gruppe: C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₂-(C₇-C₁₅-Arylalkyl), -SO₃-(C₇-C₁₅-Arylalkyl), -SO₃(C₆-C₂₄-Aryl), -SO₃(C₁-C₁₀-Alkyl), -COO-(C₆-C₂₄-Aryl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), - SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder - CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring, -NCO oder -NCS. Besonders bevorzugt steht R² für C₁-C₁₀-Alkyl, C₂-C₁₀-Alkenyl, C₃-C₁₆-Heteroaryl, C₇-C₁₅-Arylalkyl oder C₆-C₂₄-Aryl. Ganz besonders bevorzugt steht R² für Methyl, Ethyl, s-, n-Propyl, n-, i-, s-, tert.-Butyl, neo-Pentyl, cyclo-Hexyl, Benzyl, o-, m-, p-Tolyl oder Phenyl. Noch weiter bevorzugt steht R² für s-Propyl.

Me stellt bevorzugt Mg dar.

Y stellt bevorzugt C₁-C₁₀-Alkyl, F, Cl, Br oder I dar. Besonders bevorzugt steht Y für Cl oder Br.

X stellt bevorzugt Br, Cl, I oder-OSO₂-R⁸ mit R⁸ = C₁-C₁₀,-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F dar. Besonders bevorzugt steht X für Cl oder Br.

Eisenquelle stellt im Sinne der Erfindung elementares Eisen oder eine Eisenverbindung dar. Die Eisenquelle im Sinne der Erfindung dient als Katalysator. Als Eisenquelle können alle Eisenverbindungen der Oxidationsstufen -2, -1, 0, +1, +2, +3 oder elementares Eisen verwendet werden. Als Eisenverbindungen können z.B. Eisenkomplexverbindungen wie z.B., Ferrocen, Eisen(II)phtahalocyanin oder Eisenpentacarbonyl oder anorganische Eisenverbindungen wie z.B. Eisen(II)halogenide, wie z.B. Eisen(II)fluorid, Eisen(II)chlorid oder Eisen(II)bromid oder z.B. Eisen(III)halogenide wie z.B. Eisen(III)fluorid, Eisen(III)chlorid oder Eisen(III)bromid oder hydratisierte Eisen(II)- oder Eisen(III)halogenide wie z.B. Eisen(III)chlorid-Hexahydrat oder Eisen(II)chlorid-Tetrahydrat oder Eisen(II)- oder Eisen(III)-, -nitrate, -sulfate, -phosphate, -carbonate, -perchlorate oder organische Eisenverbindungen wie z.B. Eisen(II)- oder Eisen(III)-, -acetat, -formiat, -oxalat, -acetylacetonate, -stearat, -pivalat oder -gluconat oder Mischungen solcher Eisenquellen eingesetzt werden. Bevorzugt werden als Eisenverbindung elementares Eisen oder anorganische Eisenverbindungen wie bevorzugt Eisen(II)- oder Eisen(III)-, -nitrate, -sulfate, -phosphate, -carbonate, -perchlorate oder organische Eisenverbindungen wie Eisen(II)- oder Eisen(III)- ,-acetat, -formiat, -oxalat, -acetylacetonate,- stearat, -pivalat oder -gluconat oder Mischungen solcher Eisenquellen eingesetzt. Besonders bevorzugt werden als Eisenquellen Eisen(II)-oder Eisen(III)halogenide oder Eisen(III)- oder Eisen(II)acetylacetonate ganz besonders bevorzugt Eisen(III)-acetylacetonat, eingesetzt.

Alkyl beziehungsweise Alkenyl beziehungsweise Alkoxy beziehungsweise Alkynyl steht jeweils unabhängig für einen geradkettigen, zyclischen oder verzweigten Alkyl- beziehungsweise Akenylbeziehungsweise Alkoxy- beziehungsweise Alkynyl-Rest. Gleiches gilt für den nichtaromatischen Teil eines Arylalkyl-Restes.

C₁-C₁₀-Alkyl steht beispielsweise für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und 1-Ethyl-2-methylpropyl. Bevorzugt steht C₁-C₁₀-Alkyl für Methyl, Ethyl, n-Propyl, iso-Propyl, n-Butyl, sec.-Butyl, tert.-Butyl, n-Pentyl, 1-Methylbutyl, 2-Methylbutyl, 3-Methylbutyl, neo-Pentyl, 1-Ethylpropyl, cyclo-Hexyl, cyclo-Pentyl und n-Hexyl.

Beispielhaft und vorzugsweise steht C₂-C₁₀-Alkenyl für Vinyl, Allyl, Isopropenyl, cyclo-Hexenyl, cyclo-Pentenyl und n-But-2-en-1-yl.

C₁-C₁₀-Alkoxy steht beispielsweise und vorzugsweise für Methoxy, Ethoxy, n-Propoxy, iso-Propoxy, n-Butoxy, sec.-Butoxy und tert.-Butoxy, n-Pentoxy, 1-Methylbutoxy, 2-Methylbutoxy, 3-Methylbutoxy, neo-Pentoxy, 1-Ethylpropoxy, cyclo-Hexoxy, cyclo-Pentoxy und n-Hexoxy.

Beispielhaft und vorzugsweise steht C₂-C₁₀-Alkynyl für Ethinyl, Propinyl, 1-Butinyl, 2-Butinyl oder 3-Butinyl.

C₆-C₂₄-Aryl steht im Rahmen der Erfindung für einen mono-, bi- oder trizyklischen carbozyklischen aromatischen Rest mit vorzugsweise 6 bis 24 aromatischen Kohlenstoffatomen. Weiterhin können die carbozyklischen aromatischen Reste mit bis zu fünf gleichen oder verschiedenen Substituenten pro Zyklus substituiert sein, ausgewählt aus der Gruppe C₁-C₁₀-Alkyl, C₂-C₁₀-Alkenyl, C₁-C₆-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -COO-(C₆-C₂₄-Aryl),-OCOO-(C₁-C₁₀-Alkyl), -OCOO-(C₇-C₁₅-Arylalkyl), -OCOO-(C₆-C₂₄-Aryl), -SO₃-(C₇-C₁₅-Arylalkyl), -SO₃(C₆-C₂₄-Aryl), -SO₃(C₁-C₁₀-Alkyl), -SO₂-(C₇-C₁₅-Arylalkyl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder - SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring. Beispielhaft und bevorzugt steht C₆-C₂₄-Aryl für Biphenyl, Phenyl, o-, m-, p- Tolyl, Naphthyl, Phenanthrenyl, Anthracenyl, Acetnaphtylen und Fluorenyl.

C₇-C₁₅-Arylalkyl bedeutet jeweils unabhängig voneinander einen geradkettigen, zyklischen, oder verzweigten Alkyl-Rest nach vorstehender Definition, der einfach, mehrfach oder vollständig durch Aryl-Reste gemäß vorstehender Definition substituiert sein kann. Beispielsweise und vorzugsweise steht C₇-C₁₅-Arylalkyl für Benzyl, 1-Phenylethylen, 1-Phenylpropylen, 2-Phenylpropylen, 1-Phenylbutylen oder 3-Phenyl-2-methylpropylen.

3- bis 7-gliedriger gesättigter oder teilweise ungesättigter Heterozyklus steht im Rahmen der Erfindung für einen Heterozyklus, mit bis zu 3 gleichen oder verschiedenen Heteroatomen aus der Reihe S, N und/oder O, der über ein Ringkohlenstoffatom oder ein Ringstickstoffatom verknüpft ist und der eine oder zwei Doppelbindungen enthalten kann. Bevorzugt ist ein 5- bis 7-gliedriger gesättigter Heterozyklus mit bis zu 2 gleichen oder verschiedenen Heteroatomen aus der Reihe S, N und/oder O. Beispielhaft seien genannt: Tetrahydrofur-2-yl, Tetrahydrofur-3-yl, Pyrrolidin-1-yl, Pyrrolidin-2-yl, Pyrrolidin-3-yl, Pyrrolin-1-yl, Piperidin-1-yl, Piperidin-4-yl, 1,2-Dihydropyridin-1-yl, 1,4-Dihydropyridin-1-yl, Piperazin-1-yl, Morpholin-4-yl, Thiomorpholin-4-yl, Azepin-1-yl, 1,4-Diazepin-1-yl. Bevorzugt sind Piperidinyl, Piperazinyl, Morpholinyl und Pyrrolidinyl.

C₁-C₁₀-Perhalogenalkyl steht für einen vollständig durch Halogenatome substituierten C₁-C₁₀-Alkylrest darstellt. Bevorzugt steht C₁-C₁₀-Perhalogenalkyl für ein C₁-C₁₀-Perfluoralkyl. Beispielhaft und vorzugsweise steht C₁-C₁₀-Perhalogenalkyl für Trifluormethyl, Trichlormethyl, Tribrommethyl, Pentafluorethyl, Heptafluorpropyl, cylco-Nonafluorpentyl, cyclo-Nonachlorcylcopentyl, Heptafluorisopropyl und Nonafluorbutyl. Bevorzugt steht C₁-C₁₀-Perfluoralkyl für Difluormethyl, Trifluormethyl, Trichlormethyl, Tribrommethyl, Pentafluorehtyl, Heptafluorisopropyl und Nonafluorbutyl. Ganz besonders bevorzugt steht C₁-C₁₀-Perhalogenalkyl oder/und C₁-C₁₀-Perfluoralkyl für Trifluormethyl, Pentafluorethyl oder Heptafluorisopropyl.

C₃-C₁₆-Heteroaryl steht im Rahmen der Erfindung für einen aromatischen Heterozyklus, mit bis zu 3 gleichen oder verschiedenen Heteroatomen aus der Reihe S, N und/oder O, der über ein Ringkohlenstoffatom des Heteroaromaten, gegebenenfalls auch über ein Ringstickstoffatom des Heteroaromaten verknüpft ist und der zwischen 3 und 16 Kohlenstoffatome (C₃-C₁₆-Heteroaryl), vorzugsweise 3 bis 7 (C₃-C₇) Kohlenstoffatome und besonders bevorzugt 4 bis 5 (C₄-C₅) Kohlenstoffatome (C₄-C₅-Heteroaryl) aufweist. C₃-C₁₆-Heteroaryl, C₃-C₇-Heteroaryl, und C₄-C₅-Heteroaryl weisen immer mindestens so viele Heteroatome auf, dass der Heteroaromat aromatisch ist. Ein C₃-Heteroaryl weist also drei Kohlenstoffatome und mindestens zwei Stickstoffatome oder ein Stickstoffatom und ein Sauerstoffatom oder ein Stickstoffatom und ein Schwefelatom auf. C₃-C₁₆-Heteroaryl kann weiter substituiert sein durch Reste ausgewählt aus der Gruppe C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₆-C₂₄-Aryl), -COO-(C₇-C₁₅-Arylalkyl), -OCOO-(C₁-C₁₀-Alkyl), -OCOO-(C₇-C₁₅-Arylalkyl), - OCOO-(C₆-C₂₄-Aryl), -SO₃-(C₇-C₁₅-Arylalkyl), -SO₃(C₆-C₂₄-Aryl), -SO₃(C₁-C₁₀-Alkyl), -SO₂-(C₇-C₁₅-Arylalkyl), -SO₂(C₁-C₁₀-Alkyl), -SO₂(C₆-C₂₄-Aryl), -CO-(C₁-C₁₀-Alkyl), -CO-(C₆-C₂₄-Aryl), - SO₂-NH-(C₁-C₁₀-Alkyl), -SO₂-NH-(C₇-C₁₅-Arylalkyl), -SO₂NH-(C₆-C₂₄-Aryl) oder -SO₂(NR⁹) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring.. Beispielhaft und vorzugsweise seien als C₃-C₁₆-Heteroaryl genannt: Pyridyl, Pyrimidyl, Pyridazinyl, Pyrazinyl, Thienyl, Furyl, Pyrrolyl, Pyrazolyl, Imidazolyl, Thiazolyl, Oxazolyl oder Isoxazolyl, Indolizinyl, Indolyl, Benzo[b]thienyl, Benzo[b]furyl, Indazolyl, Chinolyl, Isochinolyl, Naphthyridinyl, Chinazolinyl, Benzofuranyl oder Dibenzofuranyl.

C₁-C₈-Mono- bzw. Dialkylamino bzw. steht im Rahmen der Erfindung für eine Amino-Gruppe, die mit einem oder zwei gleichen oder verschiedenen, zyklischen, geradkettigen oder verzweigten Alkylsubstituenten, die vorzugsweise jeweils 1 bis 8 Kohlenstoffatomen aufweisen.

Beispielhaft und vorzugsweise steht C₁-C₈-Monoalkylamino für Methylamino, Ethylamino, n-Propylamino, Isopropylamino, t-Butylamino, n-Pentylamino und n-Hexylamino.

Beispielhaft und vorzugsweise steht C₁-C₈-Dialkylamino für *N,N* Dimethylamino, *N,N* Diethylamino, *N-*Ethyl-*N*-methylamino, *N*-Methyl-*N*-n-propylamino, *N*-Isopropyl-*N*-n-propylamino, *N*-t-Butyl-*N-*methylamino, *N*-Ethyl-*N*-n-pentylamino und *N*-n-Hexyl-*N*-methylamino.

Der Rahmen der Erfindung erfasst alle oben stehenden und im Folgenden aufgeführten allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Das erfindungsgemäße Verfahren zur Herstellung der Verbindungen der Formel (1) kann in Gegenwart oder Abwesenheit eines Lösungsmittels durchgeführt werden. Bevorzugt wird das erfindungsgemäße Verfahren zur Herstellung der Formel (1) in Gegenwart eines organischen Lösungsmittels durchgeführt. Das erfindungsgemäße Verfahren zur Herstellung der Verbindungen der Formel (1) kann in jedem organischen, inerten Lösungsmittel durchgeführt werden. Beispielsweise und vorzugsweise können als organische Lösungsmittel lineare, zyklische und verzweigte aliphatische Kohlenwasserstoffe, wie beispielsweise, Pentan, Hexan, Heptan, Octan, iso-octan oder cyclo-Hexan oder aromatische Kohlenwasserstoffe, wie beispielsweise Benzol, Toluol, Xylol, Ethylbenzol, oder Mesitylen oder Ether wie beispielsweise 1,4-Dioxan, Tetrahydrofuran, Methyltetrahydrofuran, Dibutylether, Methyl-t-butylether, Diisopropylether, Diethylenglycol-dimethylether, Dimethoxymethan oder Amine, wie Tetramethylharnstoff, N,N,N',N'-Tetramethylethylendiamin oder Amide wie beispielsweise Dimethylformamid, Diethylformamid, N-Methylpyrrolidon, Dimethylacetamid oder Dimethylsulfoxid oder Sulfolan oder organische Carbonate wie z.B. Propylencarbonat oder Diethylcarbonat oder Gemische dieser Lösungsmittel eingesetzt werden. Besonders bevorzugt werden Ether, wie insbesondere, Dioxan, Tetrahydrofuran, tert.-Butylmethylether, Amine, wie insbesondere, N,N,N',N'-Tetramethylethylendiamin oder Amide wie insbesondere N-Methylpyrrolidon, Dimethylformamid, Diethylformamid, Dimethylacetamid oder Dimethylsulfoxid oder Sulfolan oder Mischungen dieser Lösungsmittel eingesetzt. Ganz besonders bevorzugt wird als organisches Lösungmittel N-Methylpyrrolidon eingesetzt oder ein organisches Lösungsmittelgemisch welches N-Methylpyrrolidon enthält.

Das erfindungsgemäße Verfahren zur Herstellung der Verbindungen der Formel (1) wird beispielsweise bei Temperaturen zwischen -100 °C und 50 °C, bevorzugt bei Temperaturen zwischen-50 °C und 10 °C, besonders bevorzugt bei Temperaturen zwischen -20 °C und +10°C durchgeführt.

Das erfindungsgemäße Verfahren wird in der Regel bei Standarddruck durchgeführt. Im Allgemeinen kann das Verfahren bei jedem beliebigen Druck durchgeführt werden.

Gemäß dem erfindungsgemäßen Verfahren werden die Eisenquellen in Stoffmengenverhältnissen von 50:1 bis 1:50 bezogen auf die Verbindungen der Formel (3) eingesetzt, bevorzugt werden die Eisenquellen in Stoffmengenverhältnissen von 30:1 bis 1:30 bezogen auf die Verbindungen der Formel (3) eingesetzt und besonders bevorzugt in Stoffmengenverhältnissen von 20:1 bis 1:20 bezogen auf die Verbindungen der Formel (3) eingesetzt.

In dem erfindungsgemäßen Verfahren zur Herstellung der Verbindungen der Formel (1) liegen die Stoffmengenverhältnisse der Verbindungen der Formel (2) und der Verbindungen der Formel (3) zwischen 1:5 und 5:1, bevorzugt zwischen 1:5 und 1:1 und besonders bevorzugt liegen die Stoffmengenverhältnisse der Verbindungen der Formel (2) und der Verbindungen der Formel (3) zwischen 1:3 und 1:1.

Das erfindungsgemäße Verfahren zur Herstellung der Verbindungen der Formel (I) aus den Verbindungen der Formel (II) wird im Wesentlichen wasserfrei durchgeführt. Im Wesentlichen wasserfrei bedeutet, dass der Wassergehalt bezogen auf die Menge der eingesetzten Reaktionsmischung vorzugsweise zwischen 0.0001 Gew. % und 0.1 Gew.-% liegt.

Das erfindungsgemäße Verfahren zur Herstellung der Verbindungen der Formel (1) wird vorzugsweise so durchgeführt, dass zunächst die Verbindungen der Formel (2), die Eisenquelle, und das Lösungsmittel vorgelegt werden. Danach wird die Reaktionsmischung im Allgemeinen inertisiert, durch z.B. Verdrängung der Luft mittels wasserfreiem Stickstoff oder Argon. Dann werden z.B. die Verbindungen der Formel (3), vorzugsweise dosiert, hinzugegeben. Das Ende der Reaktion kann mit dem Fachmann bekannten analytischen Verfahren, wie z.B. chromatographisch bestimmt werden. Die weitere Aufarbeitung erfolgt nach dem Fachmann bekannten und gängigen Verfahren zur Hydrolyse der Produkte aus Grignardreaktionen, durch Zugabe katalytischer Mengen an Wasser oder wasserhaltigen Verbindungen, wie z.B. gesättigte Salzlösungen und gegebenenfalls weiterer Aufreinigung z.B. mittels Extraktion mit organischen Lösungsmitteln oder/und Kristallisation. Die oben dargestellte Zugabe der Edukte kann ebenfalls in anderer Reihenfolge oder parallel erfolgen. Bevorzugt wird die Verbindung der Formel (1) durch Umkristallisation mit organischen Lösungsmitteln weiter aufgereinigt.

Die Verbindungen der Formel (1) können auch in Form Ihrer Ammoniumsalze entstehen. Die Ammoniumsalze der Verbindungen der Formel (1) können nach gängigen dem Fachmann bekannten Verfahren in die freien Verbindungen der Formel (1) z.B. und vorzugsweise mittels Umsetzung bzw. Titration mit Carbonsäuren, insbesondere mit Citronensäure, überführt werden.

Von der Erfindung ist zudem ein Verfahren zur Herstellung der Verbindungen der Formel (4) umfasst, 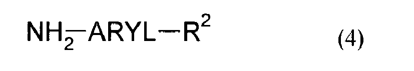 wobei ARYL und R² die vorgenannte Bedeutung haben, bei dem die Verbindungen der Formel (1) in Gegenwart mindestens einer Säure oder mindestens einer Base zu Verbindungen der Formel (4) oder deren Salze umgesetzt werden.

Basen im Sinne der Erfindung zur Herstellung der Verbindungen der Formel (4) aus den Verbindungen der Formel (1) im erfindungsgemäßen Verfahren eingesetzt werden können sind z.B. Erdalkali- oder Alkalimetallcarbonate, -hydroxide, -hydrogenphosphate, -phosphate oder tertiäre Amine.

Säuren im Sinne der Erfindung zur Herstellung der Verbindungen der Formel (4) aus den Verbindungen der Formel (1) stellen z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Citronensäsure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren oder Laurylschwefelsäure dar. Bevorzugt werden als Säuren Halogenwasserstoffsäuren, wie z.B. HCl oder HBr eingesetzt. Ganz besonders bevorzugt wird HCl als Säure im erfindungsgemäßen Verfahren eingesetzt.

Im Allgemeinen kann die Herstellung der Verbindungen der Formel (4) aus den Verbindungen der Formel (1) nach gängigen dem Fachmann bekannten Verfahren zur Entschützung von Aminen aus Amiden oder Carbamiden erfolgen.

Die Verbindung der Formel (4) können ebenfalls in Form Ihrer Ammoniumsalze vorliegen. Daher ist von der Erfindung ebenfalls ein Verfahren zur Herstellung der Verbindungen der Formel (4) umfasst, bei dem die Verbindungen der Formel (4) in Form Ihrer Ammoniumsalze vorliegen.

Die Verbindungen der Formel (2) können nach dem Fachmann bekannten Verfahren, wie z.B. in Journal of Organic Chemistry 2008, 73, 6025-6028 beschrieben ist, hergestellt werden.

Die Verbindungen der Formel (4) können ausgehend von einer Aminohalogenarylverbindung, insbesondere ausgehend von Amino-2-chlorpyridin unter Verwendung des erfindungsgemäßen Verfahrens zur Herstellung der Verbindungen der Formel (1) in einem Verfahren ausgehend von Verbindungen der Formel (5) 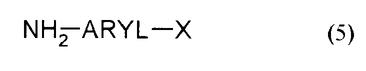 in denen ARYL die vorgenannte Bedeutung besitzt und X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy,Cl, Br oder F hergestellt werden.

Von der Erfindung ist daher ebenfalls ein Verfahren zur Herstellung der Verbindungen der Formel (4) oder deren Salze umfasst, 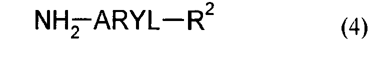 wobei ARYL für einen unsubstituierten oder substituierten, carbozyklischen C₆-C₂₄-Aryl- oder unsubstituierten oder substituierten heteroaromatischen C₃-C₁₆₋Heteroarylrest steht und

R²= C₁-C₁₀-Alkyl, C₂-C₁₀-Alkenyl, C₆-C₂₄-Aryl, C₇-C₁₅-Arylalkyl, C₃-C₁₆-Heteroaryl oder 3- bis 7- gliedriger gesättigter oder teilweise ungesättigter Heterozyklus welche gegebenenfalls weiter substituiert sein können durch Reste ausgewählt aus der Gruppe: C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₂-(C₇-C₁₅-Arylalkyl), -SO₃-(C₇-C₁₅-Arylalkyl), -SO₃(C₆-C₂₄-Aryl), -SO₃(C₁-C₁₀-Alkyl), -COO-(C₆-C₂₄-Aryl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder-SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring, bei dem in einem Schritt a) Verbindungen der Formel (5) 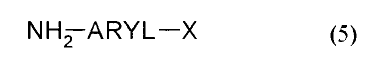 in denen ARYL die vorgenannte Bedeutung besitzt und X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F oder, mit Verbindungen der Formel (6) 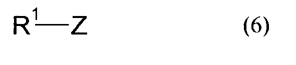 in denen R¹ = -COOR³ oder -SO₂-R⁴ wobei R³ und R⁴ gleich oder verschieden sein können und ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, Cl, Br oder F oder R¹ = - SO₂-NH-(C₁-C₁₀-Alkyl), -SO₂-NH-(C₇-C₁₅-Arylalkyl), -SO₂NH-(C₆-C₂₄-Aryl) oder -SO₂(NR⁵R⁶) wobei R⁵ und R⁶ für C₁-C₁₀-Alkyl stehen oder NR⁵R⁶ bilden zusammen einen 5- bis 7-gliedrigen Ring und
Z steht für Fluor, Chlor, Brom, Iod oder gegebenenfalls substituiertes oder unsubstituiertes -O-CO₂-(C₁-C₁₀-Alkyl), -O-CO₂(C₆-C₂₄-Aryl), -O-CO₂-(C₇-C₁₅-Arylalkyl), -OSO₂(C₁-C₁₀-Alkyl)), - OSO₂(C₆-C₂₄-Aryl), -OSO₂-NH-(C₇-C₁₅-Arylalkyl) oder -OSO₂-(C₇-C₁₅-Arylalkyl), zu Verbindungen der Formel (7) umgesetzt werden

W-ARYL-X (7)

in denen ARYL und X die vorgenannte Bedeutung besitzen und W = -NHR¹ oder -N(R¹)₂ wobei R¹ die o.g. Bedeutung besitzen und in einem Schritt b) die Verbindungen der Formel (7) mit Verbindungen der Formel (3) 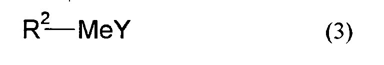 in denen R² die vorgenannte Bedeutung besitzt und Y = anionischer Ligand und Me ein Metall ausgewählt aus der Gruppe Mg, Ca, Mn, Zn ist in Gegenwart von mindestens einer Eisenquelle zu Verbindungen der Formel (1) umgesetzt werden und die Verbindungen der Formel (1) in einem Schritt c) in Gegenwart von Säuren oder Basen zu Verbindungen der Formel (4) umgesetzt werden.

Z ist bevorzugt Fluor, Chlor, Brom, Iod, -O-CO₂-(C₁-C₁₀-Alkyl), -O-CO₂(C₆-C₂₄-Aryl), -O-CO₂-(C₇-C₁₅-Arylalkyl), -OSO₂(C₁-C₁₀-Alkyl)), -OSO₂(C₆-C₂₄-Aryl). Besonders bevorzugt ist Z = -O-CO₂-(C₁-C₁₀-Alkyl) oder -O-CO₂(C₆-C₂₄-Aryl) und R¹ ist dann -COOR³ mit R³ = C₁-C₁₀-Alkyl oder C₆-C₂₄-Aryl, die einfach oder mehrfach, aber nicht vollständig substituiert sind durch C₁-C₁₀-Alkoxy, Cl, Br oder F. Ganz besonders bevorzugt ist Z = -OCOO-(tert.-Butyl).

Die Verbindungen der Formel (6) stellen bevorzugt Di-tert-Butyldicarbonat, Chlorameisensäureallylester, Chlorameisensäurebenzylester, p-, o-, m-Toluolsulfonsäurechlorid, Methansulfonsäurechlorid, Trifluormethansulfonsäurechlorid oder Ethansulfonsäurechlorid dar. Besonders bevorzugt stellt die Verbindung der Formel (6) Di-tert.-Butyldicarbonat dar.

Bevorzugt ist von der Erfindung ein Verfahren zur Herstellung der Verbindungen der Formel (8) umfasst, 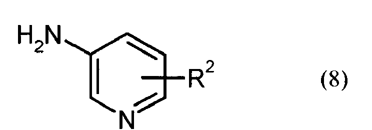 worin R² = C₁-C₁₀-Alkyl, C₂-C₁₀-Alkenyl, C₆-C₂₄-Aryl, C₇-C₁₅-Arylalkyl oder C₃-C₁₆-Heteroaryl ist und bei dem in einem Schritt a) Verbindungen der Formel (9) 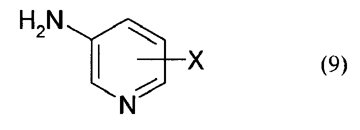 in denen X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F, mit Verbindungen der Formel (6) 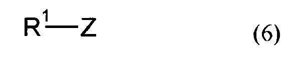 in denen R¹ = -COOR³ oder -SO₂R⁴ wobei R³ oder R⁴ gleich oder verschieden sein können und ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, Cl, Br oder F oder R¹ = - SO₂-NH-(C₁-C₁₀-Alkyl), -SO₂-NH-(C₇-C₁₅-Arylalkyl), -SO₂NH-(C₆-C₂₄-Aryl) oder -SO₂(NR⁵R⁶) wobei R⁵ und R⁶ für C₁-C₁₀-Alkyl stehen oder NR⁵R⁶ bilden zusammen einen 5- bis 7-gliedrigen Ring und

Z steht für Fluor, Chlor, Brom, Iod oder gegebenenfalls substituiertes oder unsubstituiertes -O-CO₂-(C₁-C₁₀-Alkyl), -O-CO₂(C₆-C₂₄-Aryl), -O-CO₂-(C₇-C₁₅-Arylalkyl), -OSO₂(C₁-C₁₀-Alkyl)), -OSO₂(C₆-C₂₄-Aryl), -OSO₂-NH-(C₇-C₁₅-Arylalkyl) oder -OSO₂-(C₇-C₁₅-Arylalkyl), zu Verbindungen der Formel (10) 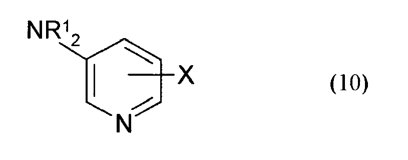 umgesetzt werden, in denen R¹ und X die vorgenannte Bedeutung besitzen und die Verbindungen der Formel (10) in einem Schritt b) mit Verbindungen der Formel (3) 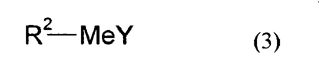 in denen R² die vorgenannte Bedeutung besitzt und Y = anionischer Ligand und Me ein Metall ausgewählt aus der Gruppe Mg, Ca, Mn, Zn ist in Gegenwart von mindestens einer Eisenquelle zu Verbindungen der Formel (11) 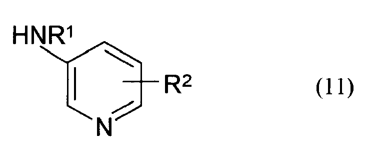 umgesetzt werden, worin R¹ und R² die vorgenannte Bedeutung besitzen und die Verbindungen der Formel (11) in einem Schritt c) in Gegenwart von Säuren oder Basen zu Verbindungen der Formel (8) oder deren Salze umgesetzt werden.

Besonders bevorzugt umfasst die Erfindung ein Verfahren zur Herstellung der Verbindungen der Formel (12) 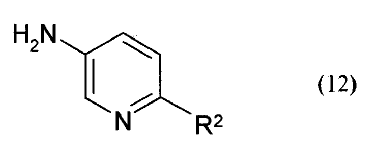 worin R² = C₁-C₁₀-Alkyl, C₂-C₁₀-Alkenyl, C₆-C₂₄-Aryl, C₇-C₁₅-Arylalkyl oder C₃-C₁₆-Heteroaryl ist und bei dem in einem Schritt a) Verbindungen der Formel (13) 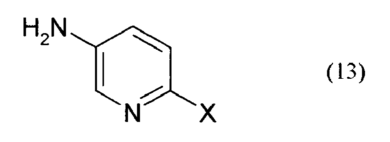 in denen X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F mit Verbindungen der Formel (14) 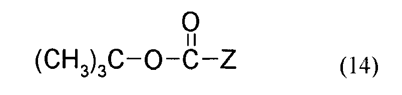 in denen Z für Fluor, Chlor, Brom, Iod oder gegebenenfalls substituiertes oder unsubstituiertes -O-CO₂-(C₁-C₁₀-Alkyl), -O-CO₂(C₆-C₂₄-Aryl), -O-CO₂-(C₇-C₁₅-Arylalkyl), -OSO₂(C₁-C₁₀-Alkyl)), -OSO₂(C₆-C₂₄-Aryl), -OSO₂-NH-(C₇-C₁₅-Arylalkyl) oder -OSO₂-(C₇-C₁₅-Arylalkyl) steht, zu Verbindungen der Formel (15) 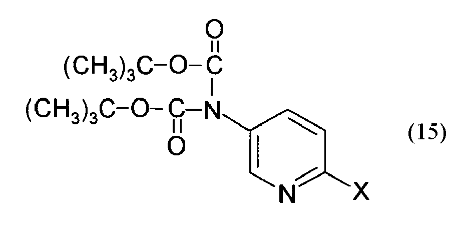 umgesetzt werden, in denen X die vorgenannte Bedeutung besitzt und die Verbindungen der Formel (15) in einem Schritt b) mit Verbindungen der Formel (3) 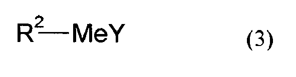 in denen R² die vorgenannte Bedeutung besitzt und Y = anionischer Ligand und Me ein Metall ausgewählt aus der Gruppe Mg, Ca, Mn, Zn ist in Gegenwart von mindestens einer Eisenquelle zu Verbindungen der Formel (16) 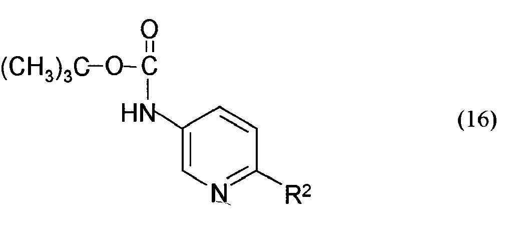 umgesetzt werden und die Verbindungen der Formel (16) in einem Schritt c) in Gegenwart von Säuren oder Basen zu Verbindungen der Formel (12) oder deren Salze umgesetzt werden.

Ganz besonders bevorzugt handelt es sich bei den Verbindungen der Formel (12) und den Verbindungen der Formel (8) und den Verbindungen der Formel (4) um 5-Amino-2-isopropylpyridin Ganz besonders bevorzugt handelt es sich bei den Verbindungen (13) und bei den Verbindungen der Formel (9) und bei den Verbindungen der Formeln (5) um 5-Amino-2-chlorpyridin.

Die Verbindungen der Formel (14) stellen bevorzugt Di-(tert.Butyl)dicarbonat dar.

Da die Herstellung der Verbindungen der Formel (8) und (12) jeweils bevorzugte Ausführungsformen der Herstellung der Verbindungen der Formel (4) darstellen, gelten die im Folgenden beschriebenen erfindungsgemäßen Verfahren gemäß der Schritte a), b) und c) für die Herstellung der Verbindungen der Formel (4), (8) und (12).

Das erfindungsgemäße Verfahren gemäß Schritt a) kann in Gegenwart von Basen oder in Abwesenheit von Basen durchgeführt werden. Bevorzugt wird das Verfahren in Abwesenheit von zusätzlichen Basen durchgeführt.

Als Basen im erfindungsgemäßen gemäß Schritt a) können beispielsweise Hydrogencarbonate, wie Natrium- und Kaliumhydrogencarbonat, Alkalimetallhydroxide oder -alkylate, wie z.B. - Natriummethylat, Kalium-tert.-butylat, Natriumhydroxid, Kaliumhydroxid oder organische Basen, wie z.B. Pyridin, Ammmonmiumverbindungen, wie z.B. Ammoniumhydroxid oder und tert. Amine, wie Trimethylamin, Triethylamin, Tributylamin, N,N-Dimethylanilin, N,N-Dimethylbenzylamin, Pyridin, N-Methylpyridin, N-Methylmorpholin, N,N-Dimethylaminopyridin, Diazabicyclooctan (DABCO), Diazabicyclononen (DBN) und Diazabicycloundecen (DBU) oder Gemische dieser Basen eingesetzt.

Das erfindungsgemäße Verfahren gemäß Schritt a) wird bevorzugt in Gegenwart von einem organischen Lösungsmittel durchgeführt werden. Als organisches Lösungsmittel zur Durchführung des erfindungsgemäßen Verfahrens eignen sich insbesondere unpolare alizyklische oder aromatische Kohlenwasserstoffe wie beispielsweise Benzol, Toluol, Xylol, n-Pentan, iso-Pentan, Hexan, Heptan, Octan, iso-Octan, Cyclohexan, Cyclopentan, Cycloheptan, Cyclononan, Cyclooctan, Methylcyclopentan, Methylcyclohexan, Bicyclo-[4.1.0]-heptan oder Gemische solcher Lösungsmittel. Besonders bevorzugte Lösungsmittel sind organische, unpolare verzweigte oder unverzweigte, gegebenenfalls cyklische, aliphatische Kohlenwasserstoffe, insbesondere Hexan, Heptan, Octan, Cyclohexan, Methylcyclohexan oder iso-Octan. Ganz besonders bevorzugt wird als Lösungsmittel n-Heptan eingesetzt.

Schritt a) des erfindungsgemäßen Verfahrens wird bei Temperaturen zwischen 20 °C und 200 °C durchgeführt. Vorzugsweise wird Schritt a) des erfindungsgemäßen Verfahrens bei Temperaturen zwischen 50 °C und 130 °C durchgeführt.

Das Stoffmengenverhältnis der Verbindungen der Formel (6) zu den Verbindungen der Formel (5) liegt zwischen 5:1 und 1:5, bevorzugt liegt das Stoffmengenverhältnis der Verbindungen der Formel (6) zu den Verbindungen der Formel (5) zwischen 3:1 und 1: 3, besonders bevorzugt zwischen 3:1 und 1:1.

Schritt a) des erfindungsgemäßen Verfahren wird vorzugsweise so durchgeführt, dass zunächst die Verbindungen der Formel (5), gegebenenfalls in Gegenwart des organischen Lösungsmittels, vorgelegt werden und dann die Verbindungen der Formel (6), bevorzugt gelöst in dem organischen Lösungsmittel, dosiert zugegeben werden. Danach wird die Mischung erwärmt. Vorzugsweise werden Teile des Lösungsmittels während der Reaktion destillativ aus dem Reaktionsgemisch entfernt. Vorzugsweise wird das Reaktionsgemisch in diesem Fall mit einer entsprechenden Menge des Lösungsmittels, gegebenenfalls und vorzugsweise Verbindungen der Formel (6) enthaltend, ersetzt. Die Verbindungen der Formel (7) können z.B. mittels Kristallisation weiter aufgereinigt werden.

Die zur Herstellung der Verbindungen der Formel (1) angegebene Verfahrensdurchführung, Temperaturen, und Vorzugsbereiche gelten ebenfalls für Schritt b) des erfindungsgemäßen Verfahrens.

Schritt c) des erfindungsgemäßen Verfahren kann in Anwesenheit oder in Gegenwart von organischen Lösungsmitteln durchgeführt werden. Vorzugsweise wird Schritt c) des erfindungsgemäßen Verfahrens in Gegenwart eines organischen Lösungsmittels durchgeführt.

Vorzugsweise werden in Schritt c) des erfindungsgemäßen Verfahrens als organische Lösungsmittel lineare, zyklische oder verzweigte aliphatische Kohlenwasserstoffe, wie beispielsweise, Pentan, Hexan, Heptan, Octan, iso-Octan oder cyclo-Hexan oder aromatische Kohlenwasserstoffe, wie beispielsweise Benzol, Toluol, Xylol, Ethylbenzol, Mesitylen oder z.B. Ketone, Alkohole, wie z.B. iso-Propanol, Ethanol, n-, s-, i-Butanol oder Sulfone oder Amide eingesetzt. Besonders bevorzugt werden als Lösungsmittel in Schritt c) des erfindungsgemäßen Verfahrens Alkohole, insbesondere iso-Propanol eingesetzt.

Basen im Sinne des Schrittes c) des erfindungsgemäßen Verfahrens sind z.B. Erdalkali- oder Alkalimetallcarbonate, -hydroxide, -hydrogenphosphate, -phosphate oder tertiäre Amine.

Säuren im Sinne des Schrittes c) des erfindungsgemäßen Verfahrens stellen z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, aromatische oder heterocyclische ein- oder mehrbasige Carbon-, Sulfonoder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Citronensäsure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono- und Disulfonsäuren oder Laurylschwefelsäure dar. Bevorzugt werden als Säuren Halogenwasserstoffsäuren, wie z.B. HCl oder HBr eingesetzt. Ganz besonders bevorzugt wird HCl als Säure im erfindungsgemäßen Verfahren eingesetzt.

Vorzugsweise wird Schritt c) des erfindungsgemäßen Verfahrens bei Temperaturen zwischen 10 °C und 100 °C, besonders bevorzugt bei Temperaturen zwischen 20 °C und 70 °C durchgeführt.

Vorzugsweise wird Schritt c) des erfindungsgemäßen Verfahrens so durchgeführt, dass die Verbindungen der Formel (1) vorgelegt werden und gegebenenfalls mit dem Lösungsmittel vermischt werden. Danach werden die Säuren oder Basen in Substanz oder als Lösung vorzugsweise dosiert hinzugegeben. Die Verbindungen der Formel (4) können z.B. durch Destillation, Kristallisation oder Extraktion weiter aufgereinigt werden. Vorzugsweise werden die Verbindungen der Formel (4) die nach der Umsetzung der Verbindungen der Formel (1) in der Regel in Form ihrer Salze vorliegen, durch Umsetzung mit Säuren oder Basen wieder in die salzfreien Verbindungen der Formel (4) überführt. Die weitere Aufreinigung der salzfreien Verbindungen der Formel (4) geschieht vorzugsweise mittels Destillation. Die weitere Aufreinigung der salzfreien Verbindungen der Formel (1) geschieht vorzugsweise mittels Destillation.

Das Ende der Reaktionen gemäß Schritt a), b) und c) des erfindungsgemäßen Verfahrens kann mit gängigen dem Fachmann bekannten Verfahren bestimmt werden.

Die in den erfindungsgemäßen Verfahren verwendeten Einsatzstoffe und Edukte sind entweder nach dem Fachmann bekannten Verfahren herstellbar oder kommerziell erhältlich.

Mit Hilfe des erfindungsgemäßen Verfahrens lassen sich die Verbindungen der Formel (1) in guten Ausbeuten, effizient in technischen Prozessen herstellen. Zudem lässt sich ebenfalls in effizienter Art und Weise aus den Verbindungen der Formel (1) durch Schutzgruppenabspaltung mit Säuren oder Basen die Verbindungen der Formel (4) herstellen, die bedeutende Zwischenprodukte bei der Herstellung von Arzneimittel darstellen.

Die folgenden Beispiele dienen der beispielhaften Erläuterung der Erfindung und sind nicht als Beschränkung aufzufassen.

### Beispiele

### 1. Herstellung von 5-(Di-Boc-amino)-2-chlorpyridin

100 g (0,76 mol) 5-Amino-2-chlorpyridin werden bei Raumtemperatur mit 120 mL (0,82 mol) n-Heptan sowie 200 g (0,89 mol) Di-*tert*.-butyl-dicarbonat versetzt. Die resultierende Suspension wird in 2 h auf 60°C aufgeheizt und für 5 h bei dieser Temperatur verrührt. Dann wird eine Lösung von 50 g (0,22 mol) Di*-tert.*-butyl-dicarbonat in 30 mL (0,20 mol) n-Heptan zugesetzt, zum Rückfluss aufgeheizt und solange Destillat abgenommen bis die Innentemperatur 115°C erreicht. Anschließend wird für 1 h bei 115°C unter Destillatabnahme nachgerührt. Nach Zugabe von 240 mL (1,64 mol) n-Heptan wird zum Rückfluss aufgeheizt und solange Destillat abgenommen bis die Innentemperatur 100°C erreicht. Dann wird unter Rückfluss eine Lösung von 200 g (0,89 mol) Di-*tert.*-butyl-dicarbonat in 120 mL (0,82 mol) n-Heptan in ca. 12 h gleichmäßig zudosiert und für weitere 4 h unter Rückfluss nachgerührt.

Der Ansatz wird langsam auf Raumtemperatur abgekühlt und für 1 h bei Raumtemperatur verrührt. Das ausgefallene Produkt wird abfiltriert und der Filterkuchen zweimal mit jeweils 120 mL (0,82 mol) n-Heptan gewaschen. Das erhaltene Feuchtprodukt wird im Vakuumtrockenschrank bei ca. 60°C und <100 mbar bis zur Gewichtskonstanz getrocknet.

Ausbeute: 241,4 g (0,73 mol, 96 %)

### 2. Herstellung von 5-(Boc-amino)-2-isopropylpyridin

Bei Raumtemperatur werden unter Stickstoff-Atmosphäre 50 g (0,15 mol) 5-(Di-Boc-amino)-2-chlorpyridin, 140 g (1,94 mol) Tetrahydrofuran sowie 10 g (0,10 mol) N-Methylpyrrolidon vorgelegt. Die resultierende Lösung wird auf ca. -20°C vorgekühlt. Anschließend wird eine Lösung von 3,0 g (8,2 mmol) Eisen(III)-acetylacetonat in 30 g (0,42 mol) Tetrahydrofuran in 3 h bei -20°C parallel mit 215 g (0,42 mol) Isopropyl-magnesiumchlorid, ca. 20%-ige Lösung in Tetrahydrofuran zudosiert. Nach Ende der Dosierung wird für 15 min bei -20°C nachgerührt, dann wird innerhalb von 1 h auf 0°C aufgeheizt und für 30 min bei 0°C nachgerührt. Der Ansatz wird anschließend auf 175 g (0,13 mol) einer 14,3%-igen Lösung von Citronensäure bei max. 10°C ausgetragen und die Reaktionsmischung für 30 min bei Raumtemperatur verrührt. Nach Phasentrennung wird die wässrige Phase einmal mit 40 g (0,13 mol) Xylol extrahiert. Die vereinigten organischen Phasen werden einmal mit 100 mL einer 5%-igen Lösung von Natriumhydrogencarbonat gewaschen und dann über 40 g (ca. 3 cm Betthöhe) Kieselgel filtriert. Anschließend wird das Filtrat im Vakuum bei 50°C bis ca. 100 mbar eingeengt. Der Destillationssumpf wird bei Raumtemperatur mit 60 g (0,61 mol) Methylcyclohexan versetzt und die resultierende Suspension auf 85°C aufgeheizt. Die resultierende klare, rot-orange Lösung wird wieder auf Raumtemperatur abgekühlt und 30 min bei Raumtemperatur nachgerührt. Das ausgefallene Produkt wird abfiltriert und der Filterkuchen einmal mit 30 g (0,31 mol) Methylcyclohexan gewaschen. Das erhaltene Feuchtprodukt wird im Vakuumtrockenschrank bei ca. 60°C und <100 mbar bis zur Gewichtskonstanz getrocknet.

Ausbeute: 25,7 g (0,11 mol, 72 %)

### 3. Herstellung von 5-Amino-2-isopropylpyridin

100 g (0,41 mol) 5-(Boc-amino)-2-isopropylpyridin werden bei Raumtemperatur in 500 g (8,3 mol) Isopropanol angeschlämmt. Dann werden 200 g (1,8 mol) einer ca. 33%-igen Lösung von Chlorwasserstoff in Isopropanol bei 25°C eindosiert. Nach Dosierende wird 30 min bei 25°C nachgerührt, dann wird in ca. 1 h auf 50°C aufgeheizt und bei 50°C für 3 h nachgerührt. Anschließend werden unter Normaldruck 600 mL Destillat abgenommen und zur resultierenden Suspension bei 40-50°C 350 g (3,8 mol) Toluol zugesetzt. Die resultierende Suspension wird bei Raumtemperatur auf eine Mischung aus 150 g (8,3 mol) VE-Wasser und 110 g (1,4 mol) Natronlauge 50%-ig ausgetragen und für 15 min bei Raumtemperatur verrührt. Nach Phasentrennung wird die organische Phase im Vakuum eingeengt und das verbleibende Öl bei 8 mbar über eine Kolonne fraktioniert destilliert.

Ausbeute: 47,7 g (0,35 mol, 85 %)

## Patentansprüche

1. Verfahren zur Herstellung der Verbindungen der Formel (1)
R¹-NH-ARYL-R² (1)
wobei R¹ = -COOR³ oder -SO₂-R⁴ wobei R³ und R⁴ ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, C1, Br oder F oder R¹ = -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁵R⁶) wobei R⁵ und R⁶ für C₁-C₁₀-Alkyl stehen oder NR⁵R⁶ bilden zusammen einen 5- bis 7-gliedrigen Ring und
ARYL für einen unsubstituierten oder substituierten carbocyclischen C₆-C₂₄-Aryl- oder unsubstituierten oder substituierten heteroaromatischen C₃-C₁₆-Heteroarylrest steht und R² = C₁-C₁₀-Alkyl, C₂-C₁₀-Alkenyl, C₆-C₂₄-Aryl, C₇-C₁₅-Arylalkyl, C₃-C₁₆-Heteroaryl oder 3- bis 7-gliedriger gesättigter oder teilweise ungesättigter Heterozyklus, welche gegebenenfalls weiter substituiert sein können durch Reste ausgewählt aus der Gruppe: C₁-C₁₀-Alkyl, C₂-C₁₀-Alkenyl, C₁-C₁₀-Alkoxy, C₁-C₁₀-Perhalogenalkyl, C₂-C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -OCOO-(C₁-C₁₀-Alkyl), -OCOO-(C₇-C₁₅-Arylalky), -OCOO-(C₆-C₂₄-Aryl), -SO₂-(C₇-C₁₅-Arylalkyl), -SO₃-(C₇-C₁₅-Arylalkyl), -SO₃(C₆-C₂₄-Aryl), -SO₃(C₁₀-C₁₀-Alkyl), -COO-(C₆-C₂₄-Aryl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂-NH-(C₁-C₁₀-Alkyl), -SO₂-NH-(C₇-C₁₅-Arylalkyl), -SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R1¹ und R¹² gleich oder verschieden sein können und unabhängig voneinander für C₁C₁₀-Alkyl oder C₆-C₂₄-Aryl stehen oder -NR¹¹R¹² bilden zusammen einen 5- bis 7- gliedrigen Ring,
**dadurch gekennzeichnet, dass** Verbindungen der Formel (2) 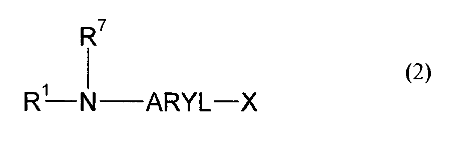 wobei R¹ und R⁷ gleich oder verschieden sein können und R¹ und ARYL die o.g. Bedeutungen besitzen und R⁷ = -COOR³ oder -SO₂-R⁴ wobei R³ und R⁴ ausgewählt sind aus der Gruppe: C₁-C₁₀-Alkyl, C₂-C₁₀-Alkenyl, C₁-C₁₀-Perhalogenalkyl, C₆-C₂₄-Aryl oder C₇-C₁₅-Arylalkyl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy, Cl. Br oder F oder R¹ = -SO₂-NH-(C₁-C₁₀-Alkyl), -SO₂-NH-(C₇-C₁₅-Arylalkyl), -SO₂NH-(C₆₋C₂₄-Aryl) oder-SO₂₍NR⁵R⁶) wobei R⁵ und R⁶ für C₁-C₁₀-Alkyl stehen oder NR⁵R⁶ bilden zusammen einen 5-bis 7-gliedrigen Ring und
X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F in Gegenwart von mindestens einer Eisenquelle
mit Verbindungen der Formel (3)
R²-MeY (3)
in denen R² die vorgenannte Bedeutung besitzt und
Y = anionischer Ligand und
Me ein Metall ausgewählt aus der Gruppe Mg, Ca, Mn, Zn ist,
zu Verbindungen der Formel (1) oder deren Salze umgesetzt werden.

2. Verfahren nach Anspruch 1, **dadurch gekennzeichnet, dass** ARYL für einen fünf- oder sechsgliedrigen heteroaromatischen Ring mit einem Stickstoffatom steht der gegebenenfalls substituiert sein kann durch 1, 2 oder 3 Substituenten ausgewählt aus der Gruppe C₁-C₁₀-Alkyl, C₁-C₁₀-Alkenyl, C₁-C₆-Halogenalkyl, C₁-C₆-Alkylthio, C₆-C₂₄-Aryl, C₃-C₁₆-Heteroaryl, 3- bis 7-gliedriger gesättigter oder teilweise ungesättigter Heterozyklus.

3. Verfahren nach Anspruch 1 oder 2, **dadurch gekennzeichnet, dass** R¹ oder/und R⁷ für -(CO)-O-(tert.Butyl), -(CO)-O-(Alkyl) -(CO)-O-(Methyl), -(CO)-O-(Ethyl), -(CO)-O-(s-Propyl), -(CO)-O-(n-Propyl), -(CO)-O-(n-Butyl), -(CO)-O-(s-Butyl), -(CO)-O-(i-Butyl), -(CO)-O-(neo-Pentyl), -(CO)-O-(Nonafluorbutyl), -(CO)-O-(Nonafluorbutyl), -SO₂-(Benzyl), -SO₂-(Dimethylbenzyl), -SO₂-(Trimethylbenzyl), -SO₂-(Phenyl), -SO₂-(o-Tolyl), -SO₂-(p-Tolyl),-SO₂-(m-Tolyl), -SO₂-(Difluorbenzyl) oder -SO₂-(Trifluorbenzyl) steht.

4. Verfahren nach einem oder mehreren der Ansprüche 1 bis 3, **dadurch gekennzeichnet, dass** R² für ein C₁-C₁₀-Alkyl, C₂-C₁₀-Alkenyl, C₃-C₁₆-Heteroaryl, C₇-C₁₅-Arylalkyl oder C₆-C₂₄-Aryl steht.

5. Verfahren nach einem oder mehreren der Ansprüche 1 bis 4, **dadurch gekennzeichnet, dass** Me für Magnesium steht.

6. Verfahren nach einem oder mehreren der Ansprüche 1 bis 5, **dadurch gekennzeichnet, dass** Y für Chlorid, Bromid oder Iodid steht.

7. Verfahren nach einem oder mehreren der Ansprüche 1 bis 6, **dadurch gekennzeichnet, dass** X für Cl. Br, I, -OSO₂(C₁-C₁₀-Alkyl)), -OSO₂(C₆-C₂₄-Aryl) oder -OSO₂-(C₇-C₁₅-Arylalkyl) steht.

8. Verfahren nach einem oder mehreren der Ansprüche 1 bis 7, **dadurch gekennzeichnet, dass** als Eisenquellen organische Eisenverbindungen der Oxidationsstufe (III) eingesetzt werden.

9. Verfahren zur Herstellung der Verbindungen der Formel (4) 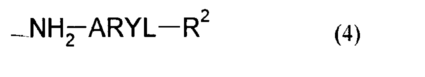 wobei ARYL und R² die unter Anspruch 1 genannte Bedeutung haben, **dadurch gekennzeichnet, dass** die Verbindungen der Formel (1) gemäß einem oder mehreren der Ansprüche 1 bis 7 hergestellt wurden und in Gegenwart einer Säure oder einer Base zu Verbindungen der Formel (4) oder deren Salze umgesetzt werden.

10. Verfahren nach Anspruch 9, **dadurch gekennzeichnet**, das als Säuren Halogenwasserstoffsäure, Sulfonsäuren oder Carbonsäuren eingesetzt werden.

11. Verfahren zur Herstellung der Verbindungen der Formel (4) oder deren Salze 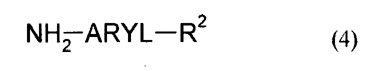 wobei ARYL für einen unsubstituierten oder substituierten, carbocyclischen C₆-C₂₄-Aryl- oder unsubstituierten oder substituierten heteroaromatischen C₃-C₁₆-Heteroarylrest steht und
R² = C₁-C₁₀-Alkyl, C₂-C₁₀-Alkenyl, C₆-C₂₄-Aryl, C₇-C₁₅-Arylalkyl, C₃-C₁₆-Heteroaryl oder 3- bis 7-gliedriger gesättigter oder teilweise ungesättigter Heterozyklus, welche gegebenenfalls weiter substituiert sein können durch Reste ausgewählt aus der Gruppe: C₁-C₁₀-Alkyl, C₂-C₁₀-Alkenyl, C₁-C₁₀-Alkoxy, C₁-C₁₀-Perhalogenalkyl, C₂C₁₀-Alkynyl, C₆-C₂₄-Aryl, C₃-C₁₆-Heteroaryl, -COO-(C₁-C₁₀-Alkyl), -COO-(C₇-C₁₅-Arylalkyl), -OCOO-(C₁-C₁₀-Alkyl), -OCOO-(C₇-C₁₅-Arylalkyl), -OCOO-(C₆-C₂₄-Aryl), -SO₂-(C₇C₁₅-Arylalkyl), -SO₃-(C₇-C₁₅-Arylalkyl), -SO₃(C₆-C₂₄-Aryl), -SO₃(C₁-C₁₀-Alkyl), -COO-(C₆-C₂₄-Aryl), -SO₂(C₁-C₁₀-Alkyl), -SO₂(C₆-C₂₄-Aryl), -CO-(C₁-C₁₀-Alkyl), -CO-(C₆-C₂₄-Aryl), -SO₂NH-(C₁-C₁₀-Alkyl), -SO₂-NH-(C₇-C₁₅-Arylalkyl), -SO₂NH-(C₆-C₂₄-Aryl) oder -SO₂(NR⁹R¹⁰) wobei R⁹ und R¹⁰ gleich oder verschieden sein können und unabhängig voneinander für C₁-C₁₀-Alkyl oder C₆-C₂₄-Aryl stehen oder NR⁹R¹⁰ bilden zusammen einen 5- bis 7- gliedrigen Ring, C₁-C₈-Mono- bzw. Dialkylamino, Halogen, -OCO-(NR¹¹R¹²) oder -CO-(NR¹¹R¹²) wobei R¹¹ und R¹² gleich oder verschieden sein können und unabhängig voneinander für C₁-C₁₀-Alkyl oder C₆-C₂₄-Aryl stehen oder -NR¹¹R¹² bilden zusammen einen 5- bis 7- gliedrigen Ring,
**dadurch gekennzeichnet, dass** in einem Schritt a) Verbindungen der Formel (5) 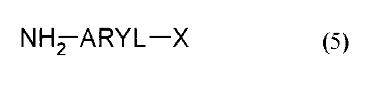 in denen ARYL die vorgenannte Bedeutung besitzt und X = Cl, Br, I oder -OSO₂-R⁸ mit R⁸ = C₁-C₁₀-Alkyl, C₁-C₁₀-Perhalogenalkyl, C₇-C₁₅-Arylalkyl oder C₆-C₂₄-Aryl oder R⁸ = C₁-C₁₀-Alkyl oder C₆-C₂₄-Aryl die einfach oder mehrfach, aber nicht vollständig substituiert sind durch C₁-C₁₀-Alkoxy, Cl, Br oder F,
mit Verbindungen der Formel (6)
R¹-Z (6)
in denen R¹ = -COOR³ oder -SO₂-R⁴ wobei R³ und R⁴ gleich oder verschieden sein können und ausgewählt sind aus der Gruppe: C₁-C₁₀-Alkyl, C₂-C₁₀-Alkenyl, C₁-C₁₀-Perhalogenalkyl, C₇-C₁₅-Arylalkyl oder C₆-C₂₄-Aryl oder R³ oder R⁴ = C₁-C₁₀-Alkyl oder C₆-C₂₄-Aryl die einfach oder mehrfach, aber nicht vollständig substituiert sind durch C₁-C₁₀-Alkoxy-, Cl, Br oder F oder R¹ = -SO₂-NH-(C₁-C₁₀-Alkyl), -SO₂-NH-(C₇-C₅-Arylalkyl), -SO₂NH-(C₆-C)₄-Aryl) oder -SO₂(NR⁵R⁶) wobei R⁵ und R⁶ für C₁-C₁₀-Alkyl stehen oder NR⁵R⁶ bilden zusammen einen 5- bis 7- gliedrigen Ring und
Z für Fluor, Chlor, Brom, Iod oder gegebenenfalls substituiertes oder unsubstituiertes -0-CO₂-(C₁C₁₀-Alkyl), -O-CO₂(C₆-C₂₄-Aryl), -O-CO₂-(C₇-C₁₅-Arylalkyl), -OSO₂(C₁-C₁₀-Alkyl)), -OSO₂(C₆-C₂₄-Aryl), -OSO₂-NH-(C₇-C₅-Arylalkyl) oder -OSO₂-(C₇-C₁₅-Arylalkyl) steht, zu Verbindungen der Formel (7) umgesetzt werden
W-ARYL-X (7)
in denen ARYL und X die vorgenannte Bedeutung besitzen und W = -NHR¹ oder -N(R¹)₂ wobei R¹ die o.g. Bedeutung besitzt und
in einem Schritt b) die Verbindungen der Formel (7) mit Verbindungen der Formel (3) 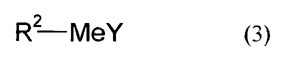 in denen R² die vorgenannte Bedeutung besitzt und Y = anionischer Ligand und Me ein Metall ausgewählt aus der Gruppe Mg, Ca, Mn, Zn ist,
in Gegenwart von mindestens einer Eisenquelle zu Verbindungen der Formel (1) umgesetzt werden und die Verbindungen der Formel (1)
in einem Schritt c) in Gegenwart von Säuren oder Basen zu Verbindungen der Formel (4) umgesetzt werden.

12. Verfahren gemäß einem oder mehreren der Ansprüche 1 bis 11, **dadurch gekennzeichnet, dass** das Verfahren gemäß Schritt b) in Gegenwart eines organischen Lösungsmittels durchgeführt wird.

13. Verfahren gemäß Anspruch 12, **dadurch gekennzeichnet, dass** das organische Lösungsmittel ausgewählt ist aus der Gruppe Ether, Amine, Amide oder Mischungen dieser Lösungsmittel.

14. Verfahren gemäß Anspruch 11, **dadurch gekennzeichnet, dass** die Verbindungen der Formel (6) Di-tert.butyldicarbonat darstellen.

## Claims

1. Process for preparing the compounds of formula (1)
R¹-NH-ARYL-R² (1)
where R¹ is -COOR³ or -SO₂-R⁴, where R³ and R⁴ are each selected from the group: C₁-C₁₀-alkyl, C₂-C₁₀-alkenyl, C₁-C₁₀-perhaloalkyl, C₇-C₁₅-arylalkyl or C₆-C₂₄-aryl, or R³ or R⁴ is C₁-C₁₀-alkyl or C₆-C₂₄-aryl each singly or multiply but not wholly substituted by C₁-C₁₀-alkoxy, Cl, Br or F, or R¹ is -SO₂-NH-(C₁-C₁₀-alkyl), -SO₂-NH-(C₇-C₁₅-arylalkyl), -SO₂NH-(C₆-C₂₄-aryl) or -SO₂(NR⁵R⁶), where R⁵ and R⁶ each represent C₁-C₁₀-alkyl, or NRSR⁶ together form a 5-to 7-membered ring, and
ARYL represents a substituted or unsubstituted carbocyclic C₆-C₂₄-aryl radical or a substituted or unsubstituted heteroaromatic C₃-C₁₆-hetaryl radical, and
R² is C₁-C₁₀-alkyl, C₂-C₁₀-alkenyl, C₆-C₂₄-aryl, C₇-C₁₅-arylalkyl, C₃-C₁₆-hetaryl or a 3- to 7-membered saturated or partially unsaturated heterocycle which may optionally be further substituted by radicals selected from the group: C₁-C₁₀-alkyl, C₂-C₁₀-alkenyl, C₁-C₁₀-alkoxy, C₁-C₁₀-perhaloalkyl, C₂-C₁₀-alkynyl, C₆-C₂₄-aryl, C₃-C₁₆-hetaryl, -COO-(C₁-C₁₀-alkyl), -COO-(C₇-C₁₅-arylalkyl), -OCOO-(C₁-C₁₀-alkyl), -OCOO-(C₇-C₁₅-arylalkyl), -OCOO-(C₆-C₂₄-aryl), -SO₂-(C₇-C₁₅-arylalkyl), -SO₃-(C₇-C₁₅-arylalkyl), -SO₃(C₆-C₂₄-aryl), -SO₃(C₁-C₁₀-alkyl),-COO-(C₆-C₂₄-aryl), -SO₂(C₁-C₁₀-alkyl), -SO₂(C₆-C₂₄-aryl), -CO-(C₁-C₁₀-alkyl), -CO-(C₆-C₂₄-aryl), -SO₂-NH-(C₁-C₁₀-alkyl), -SO₂-NH-(C₇-C₁₅-arylalkyl), -SO₂NH-(C₆-C₂₄-aryl) or SO₂(NR⁹R¹⁰), where R⁹ and R¹⁰ may be the same or different and each independently represent C₁-C₁₀-alkyl or C₆-C₂₄-aryl or NR⁹R¹⁰ together form a 5- to 7-membered ring, C₁-C₈-mono- or -dialkylamino, halogen, -OCO-(NR¹¹R¹²) or -CO-(NR¹¹R¹²) where R¹¹ and R¹² may be the same or different and each independently represent C₁-C₁₀-alkyl or C₆-C₂4-aryl, or -NR¹¹R¹² together form a 5- to 7-membered ring,
**characterized in that** compounds of formula (2) 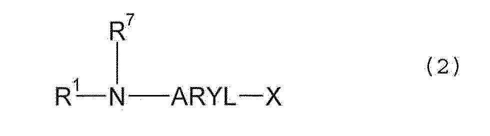 where R¹ and R⁷ may be the same or different and R¹ and ARYL are each as defined above and R⁷ is -COOR³ or -SO₂-R⁴, where R³ and R⁴ are each selected from the group: C₁-C₁₀-alkyl, C₂-C₁₀-alkenyl, C₁-C₁₀-perhaloalkyl, C₆-C₂₄-aryl or C₇-C₁₅-arylalkyl or R³ or R⁴ is C₁-C₁₀-alkyl or C₆-C₂₄-aryl each singly or multiply but not wholly substituted by C₁-C₁₀-alkoxy, Cl, Br or F, or R¹ is -SO₂-NH-(C₁-C₁₀-alkyl), -SO₂-NH-(C₇-C₁₅-arylalkyl), -SO₂NH-(C₆-C₂₄-aryl) or -SO₂(NR⁵R⁶), where R⁵ and R⁶ each represent C₁-C₁₀-alkyl, or NR⁵R⁶ together form a 5- to 7-membered ring, and
X is Cl, Br, I or -OSO₂-R⁸ where R⁸ is C₁-C₁₀-alkyl, C₁-C₁₀-perhaloalkyl, C₇-C₁₅-arylalkyl or C₆-C₂₄-aryl or R⁸ is C₁-C₁₀-alkyl or C₆-C₂₄-aryl each singly or multiply but not wholly substituted by C₁-C₁₀-alkoxy, Cl, Br or F
are reacted in the presence of at least one iron source
with compounds of formula (3) 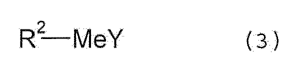 where R² is as defined above and
Y is an anionic ligand and
Me is a metal selected from the group Mg, Ca, Mn, Zn,
to form compounds of formula (1) or salts thereof.

2. Process according to Claim 1, **characterized in that** ARYL represents a five- or six-membered heteroaromatic ring which has a nitrogen atom and may be optionally substituted by 1, 2 or 3 substituents selected from the group C₁-C₁₀-alkyl, C₁-C₁₀-alkenyl, C₁-C₆-haloalkyl, C₁-C₆-alkylthio, C₆-C₂₄-aryl, C₃-C₁₆-hetaryl, 3- to 7-membered saturated or partially unsaturated heterocycle.

3. Process according to Claim 1 or 2, **characterized in that** R¹ or/and R⁷ represents - (CO)-O-(tert-butyl), -(CO)-O-(allyl), -(CO)-O-(methyl), -(CO)-O-(ethyl), -(CO)-O-(s-propyl), -(CO)-O-(n-propyl), -(CO)-O-(n-butyl), -(CO)-O-(s-butyl), -(CO)-O-(i-butyl), -(CO)-O-(neopentyl), -(CO)-O-(nonafluorobutyl), -(CO)-O-(nonafluorobutyl), -SO₂-(benzyl), -SO₂-(dimethylbenzyl), -SO₂-(trimethylbenzyl), -SO₂-(phenyl), -SO₂-(o-tolyl), - SO₂-(p-tolyl), -SO₂-(m-tolyl), -SO₂-(difluorobenzyl) or -SO₂-(trifluorobenzyl).

4. Process according to one or more of Claims 1 to 3, **characterized in that** R² represents C₁,-C₁₀-alkyl, C₂-C₁₀-alkenyl, C₃-C₁₆-hetaryl, C₇-C₁₅-arylalkyl or C₆-C₂₄-aryl.

5. Process according to one or more of Claims 1 to 4, **characterized in that** Me represents magnesium.

6. Process according to one or more of Claims 1 to 5, **characterized in that** Y represents chloride, bromide or iodide.

7. Process according to one or more of Claims 1 to 6, **characterized in that** X represents Cl, Br, I, OSO₂ (C₁-C₁₀-alkyl) -OSO₂ (C₆-C₂₄-aryl) or -OSO₂-(C₇-C₁₅-arylalkyl).

8. Process according to one or more of Claims 1 to 7, **characterized in that** organic iron compounds of oxidation state (III) are used as iron sources.

9. Process for preparing the compounds of formula (4) 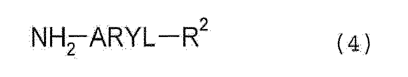 where ARYL and R² are each as defined under Claim 1, **characterized in that** the compounds of formula (1) are prepared according to one or more of Claims 1 to 7 and are reacted in the presence of an acid or a base to form compounds of formula (4) or salts thereof.

10. Process according to Claim 9, **characterized in that** hydrohalic acid, sulphonic acids or carboxylic acids are used as acids.

11. Process for preparing the compounds of formula (4) or salts thereof 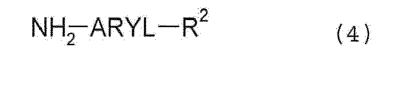 where ARYL represents a substituted or unsubstituted carbocyclic C₆-C₂₄-aryl radical or a substituted or unsubstituted heteroaromatic C₃-C₁₆-hetaryl radical, and
R² is C₁-C₁₀-alkyl, C₂-C₁₀-alkenyl, C₆-C₂₄-aryl, C₇-C₁₅-arylalkyl, C₃-C₁₆-hetaryl or a 3- to 7-membered saturated or partially unsaturated heterocycle which may optionally be further substituted by radicals selected from the group: C₁,-C₁₀-alkyl, C₂-C₁₀-alkenyl, C₁-C₁₀-alkoxy, C₁-C₁₀-perhaloalkyl, C₂-C₁₀-alkynyl, C₆-C₂₄-aryl, C₃-C₁₆-hetaryl, -COO-(C₁-C₁₀-alkyl), -COO-(C₇-C₁₅-arylalkyl), -OCOO-(C₁-C₁₀-alkyl), -OCOO-(C₇-C₁₅-arylalkyl), -OCOO-(C₆-C₂₄-aryl), -SO₂-(C₇-C₁₅-arylalkyl), -SO₃-(C₇-C₁₅-arylalkyl), -SO₃(C₆-C₂₄-aryl), -SO₃(C₁-C₁₀-alkyl), - COO-(C₆-C₂₄-aryl), -SO₂(C₁-C₁₀-alkyl), -SO₂(C₆-C₂₄-aryl), -CO-(C₁-C₁₀-alkyl), -CO-(C₆-C₂₄-aryl), -SO₂-NH-(C₁-C₁₀-alkyl), -SO₂-NH-(C₇-C₁₅-arylalkyl) -SO₂NH-(C₆-C₂₄-aryl) or -SO₂(NR⁹R¹⁰), where R⁹ and R¹⁰ may be the same or different and each independently represent C₁-C₁₀-alkyl or C₆-C₂₄-aryl or NR⁹R¹⁰ together form a 5- to 7-membered ring, C₁-C₈-mono- or -dialkylamino, halogen, -OCO-(NR¹¹R¹²) or -CO-(NR¹¹R¹²), where R¹¹ and R¹² may be the same or different and each independently represent C₁-C₁₀-alkyl or C₆-C₂₄-aryl, or -NR¹¹R¹² together form a 5- to 7-membered ring,
**characterized in that** in a step a) compounds of formula (5) 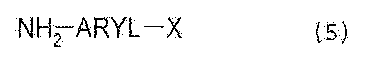 where ARYL is as defined above and X is Cl, Br, I or -OSO₂-R⁸ where R⁸ is C₁-C₁₀-alkyl, C₁-C₁₀-perhaloalkyl, C₇-C₁₅-arylalkyl or C₆-C₂₄-aryl or R⁸ is C₁-C₁₀-alkyl or C₆-C₂₄-aryl each singly or multiply but not wholly substituted by C₁-C₁₀-alkoxy, Cl, Br or F,
are reacted with compounds of formula (6)
R¹-Z (6)
where R¹ is -COOR³ or -SO₂-R⁴, where R³ and R⁴ may be the same or different and are each selected from the group: C₁-C₁₀-alkyl, C₂-C₁₀-alkenyl, C₁-C₁₀-perhaloalkyl, C₇-C₁₅-arylalkyl or C₆-C₂₄-aryl, or R³ or R⁴ is C₁-C₁₀-alkyl or C₆-C₂₄-aryl each singly or multiply but not wholly substituted by C₁-C₁₀-alkoxy, Cl, Br or F, or R¹ is -SO₂-NH-(C₁-C₁₀-alkyl), -SO₂-NH-(C₇-C₁₅-arylalkyl), -SO₂NH-(C₆-C₂₄-aryl) or -SO₂(NR⁵R⁶), where R⁵ and R⁶ each represent C₁-C₁₀-alkyl, or NR⁵R⁶ together form a 5- to 7-membered ring, and
Z represents fluorine, chlorine, bromine, iodine or optionally substituted or unsubstituted -O-CO₂-(C₁-C₁₀-alkyl), -O-CO₂ (C₆-C₂₄-aryl), -O-CO₂-(C₇-C₁₅-arylalkyl), -OSO₂(C₁-C₁₀-alkyl), -OSO₂(C₆-C₂₄-aryl), -OSO₂-NH-(C₇-C₁₅-arylalkyl) or -OSO₂-(C₇-C₁₅-arylalkyl) to form compounds of formula (7)
W-ARYL-X (7)
where ARYL and X are each as defined above and W is -NHR¹ or -N(R¹)₂, where R¹ is as defined above, and in a step b) the compounds of formula (7) are reacted with compounds of formula (3) 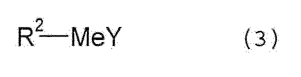 where R² is as defined above and Y is an anionic ligand and Me is a metal selected from the group Mg, Ca, Mn, Zn,
in the presence of at least one iron source to form compounds of formula (1), and
in a step c) the compounds of formula (1) are reacted in the presence of acids or bases to form compounds of formula (4).

12. Process according to one or more of Claims 1 to 11, **characterized in that** the process according to step b) is carried out in the presence of an organic solvent.

13. Process according to Claim 12, **characterized in that** the organic solvent is selected from the group ethers, amines, amides or mixtures thereof.

14. Process according to Claim 11, **characterized in that** the compounds of formula (6) are di-tert-butyl dicarbonate.

## Revendications

1. Procédé de fabrication des composés de formule (1)
R¹-NH-ARYL-R² (1)
avec R¹ = -COOR³ ou -SO₂-R⁴, R³ et R⁴ étant choisis dans le groupe : alkyle en C₁-C₁₀, alcényle en C₂-C₁₀, perhalogénoalkyle en C₁-C₁₀, arylalkyle en C₇-C₁₅ ou aryle en C₆-C₂₄, ou R³ ou R⁴ = alkyle en C₁-C₁₀ ou aryle en C₆-C₂₄, qui sont substitués une ou plusieurs fois, mais pas en totalité, par alcoxy en C₁-C₁₀, Cl, Br ou F, ou R¹ = - SO₂-NH- (alkyle en C₁-C₁₀), -SO₂-NH- (arylalkyle en C₇-C₁₅), -SO₂-NH-(aryle en C₆-C₂₄) ou SO₂-(NR⁵R⁶), R⁵ et R⁶ représentant alkyle en C₁-C₁₀ ou NR⁵R⁶ formant ensemble un cycle de 5 à 7 éléments, et
ARYLE représentant un radical aryle en C₆-C₂₄ carbocyclique non substitué ou substitué ou un radical hétéroaryle en C₃-C₁₆ hétéroaromatique non substitué ou substitué, et
R² = alkyle en C₁-C₁₀, alcényle en C₂-C₁₀, aryle en C₆-C₂₄, arylalkyle en C₇-C₁₅, hétéroaryle en C₃-C₁₆ ou un hétérocycle saturé ou partiellement insaturé de 3 à 7 éléments, qui peuvent éventuellement être davantage substitués par des radicaux choisis dans le groupe : alkyle en C₁-C₁₀, alcényle en C₂-C₁₀, alcoxy en C₁-C₁₀, perhalogénoalkyle en C₁-C₁₀, alcynyle en C₂-C₁₀, aryle en C₆-C₂₄, hétéroaryle en C₃-C₁₆, -COO-(alkyle en C₁-C₁₀), - COO-(arylalkyle en C₇-C₁₅), -OCOO-(alkyle en C₁-C₁₀), - OCOO-(arylalkyle en C₇-C₁₅), -OCOO-(aryle en C₆-C₂₄), -SO₂-(arylalkyle en C₇-C₁₅), -SO₃-(arylalkyle en C₇-C₁₅), -SO₃-(aryle en C₆-C₂₄), -SO₃-(alkyle en C₁-C₁₀), -COO-(aryle en C₆-C₂₄), -SO₂-(alkyle en C₁-C₁₀), -SO₂-(aryle en C₆-C₂₄), - CO-(alkyle en C₁-C₁₀), -CO-(aryle en C₆-C₂₄), -SO₂-NH-(alkyle en C₁-C₁₀), -SO₂-NH- (arylalkyle en C₇-C₁₅) -SO₂-NH- (aryle en C₆-C₂₄) ou -SO₂-(NR⁹R¹⁰), R⁹ et R¹⁰ pouvant être identiques ou différents et représentant indépendamment l'un de l'autre alkyle en C₁-C₁₀ ou aryle en C₆-C₂₄, ou NR⁹R¹⁰ formant ensemble un cycle de 5 à 7 éléments, mono- ou dialkylamino en C₁-C₈, halogène, - OCO-(NR¹¹R¹²) ou -CO-(NR¹¹R¹²), R¹¹ et R¹² pouvant être identiques ou différents et représentant indépendamment l'un de l'autre alkyle en C₁-C₁₀ ou aryle en C₆-C₂₄, ou - NR¹¹R¹² formant ensemble un cycle de 5 à 7 éléments,
**caractérisé en ce que** des composés de formule (2) 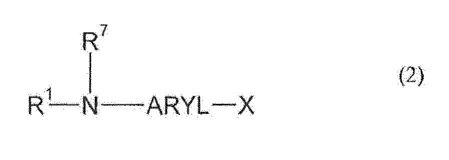 dans laquelle R¹ et R⁷ peuvent être identiques ou différents et R¹ et ARYL ont les significations indiquées précédemment et R⁷ = -COOR³ ou -SO₂-R⁴, R³ et R⁴ étant choisis dans le groupe : alkyle en C₁-C₁₀, alcényle en C₂-C₁₀, perhalogénoalkyle en C₁-C₁₀, aryle en C₆-C₂₄ ou arylalkyle en C₇-C₁₅, ou R³ ou R⁴ = alkyle en C₁-C₁₀ ou aryle en C₆-C₂₄, qui sont substitués une ou plusieurs fois, mais toutefois pas en totalité, par alcoxy en C₁-C₁₀, Cl, Br ou F, ou R¹ = -SO₂-NH- (alkyle en C₁-C-₁₀), -SO₂-NH- (arylalkyle en C₇-C₁₅), -SO₂-NH-(aryle en C₆-C₂₄) ou - SO₂-(NR⁵R⁶), R⁵ et R⁶ représentant alkyle en C₁-C₁₀, ou NR⁵R⁶ formant ensemble un cycle de 5 à 7 éléments, et X = Cl, Br, I ou -OSO₂-R⁸ avec R⁸ = alkyle en C₁-C₁₀, perhalogénoalkyle en C₁-C₁₀, arylalkyle en C₇-C₁₅, ou aryle en C₆-C₂₄, ou R⁸ = alkyle en C₁-C₁₀ ou aryle en C₆-C₂₄, qui sont substitués une ou plusieurs fois, mais toutefois pas en totalité, par alcoxy en C₁-C₁₀, Cl, Br ou F, sont mis en réaction en présence d'au moins une source de fer
avec des composés de formule (3)
R²-MeY (3)
dans laquelle R² a la signification précédente et
Y = ligand anionique et
Me est un métal choisi dans le groupe Mg, Ca, Mn, Zn,
pour former des composés de formule (1) ou leurs sels.

2. Procédé selon la revendication 1, **caractérisé en ce qu'**ARYL représente un cycle hétéroaromatique de cinq ou six éléments contenant un atome d'azote, qui peut éventuellement être substitué par 1, 2 ou 3 substituants choisis dans le groupe constitué par alkyle en C₁-C₁₀, alcényle en C₁-C₁₀, halogénoalkyle en C₁-C₆, alkylthio en C₁-C₆, aryle en C₆-C₂₄, hétéroaryle en C₃-C₁₆, un hétérocycle saturé ou partiellement insaturé de 3 à 7 éléments.

3. Procédé selon la revendication 1 ou 2, **caractérisé en ce que** R¹ et/ou R⁷ représentent -(CO)-O-(tert-butyle), -(CO)-O-(allyle) (CO)-O-(méthyle), -(CO)-O-(éthyle), - (CO)-O-(s-propyle), -(CO)-O-(n-propyle), -(CO)-O-(n-butyle), -(CO)-O-(s-butyle), -(CO)-O-(i-butyle), -(CO)-O-(néo-pentyle), -(CO)-O-(nonafluorobutyle), -(CO)-O-(nonafluorobutyle), -SO₂-(benzyle), -SO₂-(diméthylbenzyle), -SO₂-(triméthylbenzyle), -SO₂-(phényle), -SO₂-(o-tolyle), -SO₂- (p-tolyle), -SO₂-(m-tolyle), -SO₂-(difluorobenzyle) ou -SO₂-(trifluorobenzyle).

4. Procédé selon une ou plusieurs des revendications 1 à 3, **caractérisé en ce que** R² représente un alkyle en C₁-C₁₀, alcényle en C₂-C₁₀, hétéroaryle en C₃-C₁₆, arylalkyle en C₇-C₁₅ ou aryle en C₆-C₂₄.

5. Procédé selon une ou plusieurs des revendications 1 à 4, **caractérisé en ce que** Me représente le magnésium.

6. Procédé selon une ou plusieurs des revendications 1 à 5, **caractérisé en ce qu'**Y représente chlorure, bromure ou iodure.

7. Procédé selon une ou plusieurs des revendications 1 à 6, **caractérisé en ce que** X représente Cl, Br, I, -OSO₂-(alkyle en C₁-C₁₀), -OSO₂- (aryle en C₆-C₂₄) ou -OSO₂-(arylalkyle en C₇-C₁₅).

8. Procédé selon une ou plusieurs des revendications 1 à 7, **caractérisé en ce que** des composés organiques de fer de niveau d'oxydation (III) sont utilisés en tant que sources de fer.

9. Procédé de fabrication des composés de formule (4)
-NH₂-ARYL-R² (4)
dans laquelle ARYL et R² ont la signification indiquée dans la revendication 1, **caractérisé en ce que** les composés de formule (1) selon une ou plusieurs des revendications 1 à 7 sont fabriqués et mis en réaction en présence d'un acide ou d'une base pour former des composés de formule (4) ou leurs sels.

10. Procédé selon la revendication 9, **caractérisé en ce que** des acides halogénohydriques, des acides sulfoniques ou des acides carboxyliques sont utilisés en tant qu'acides.

11. Procédé de fabrication des composés de formule (4) ou leurs sels
NH₂-ARYL-R² (4)
dans laquelle ARYL représente un radical aryle en C₆-C₂₄ carbocyclique non substitué ou substitué ou un radical hétéroaryle en C₃-C₁₆ hétéroaromatique non substitué ou substitué, et
R² = alkyle en C₁-C₁₀, alcényle en C₂-C₁₀, aryle en C₆-C₂₄, arylalkyle en C₇-C₁₅, hétéroaryle en C₃-C₁₆ ou un hétérocycle saturé ou partiellement insaturé de 3 à 7 éléments, qui peuvent éventuellement être davantage substitués par des radicaux choisis dans le groupe : alkyle en C₁-C₁₀, alcényle en C₂-C₁₀, alcoxy en C₁-C₁₀, perhalogénoalkyle en C₁-C₁₀, alcynyle en C₂-C₁₀, aryle en C₆-C2,₄, hétéroaryle en C₃-C₁₆, -COO-(alkyle en C₁-C₁₀), - COO-(arylalkyle en C₇-C₁₅), -OCOO-(alkyle en C₁-C₁₀), - OCOO-(arylalkyle en C₇-C₁₅), -OCOO-(aryle en C₆-C₂₄), -SO₂-(arylalkyle en C₇-C₁₅), -SO₃- (arylalkyle en C₇-C₁₅), -SO₃-(aryle en C₆-C₂₄), -SO₃-(alkyle en C₁-C₁₀), -COO-(aryle en C₆-C₂₄), -SO₂-(alkyle en C₁-C₁₀), -SO₂-(aryle en C₆-C₂₄), - CO-(alkyle en C₁-C₁₀), -CO-(aryle en C₆-C₂₄), -SO₂-NH-(alkyle en C₁-C₁₀), -SO₂-NH-(arylalkyle en C₇-C₁₅), -SO₂-NH- (aryle en C₆-C₂₄) ou -SO₂-(NR⁹R¹⁰), R⁹ et R¹⁰ pouvant être identiques ou différents et représentant indépendamment l'un de l'autre alkyle en C₁-C₁₀ ou aryle en C₆-C₂₄, ou NR⁹R¹⁰ formant ensemble un cycle de 5 à 7 éléments, mono- ou dialkylamino en C₁-C₈, halogène, - OCO-(NR¹¹R¹²) ou -CO-(NR¹¹R¹²), R¹¹ et R¹² pouvant être identiques ou différents et représentant indépendamment l'un de l'autre alkyle en C₁-C₁₀ ou aryle en C₆-C₂₄, ou - NR¹¹R¹² formant ensemble un cycle de 5 à 7 éléments, **caractérisé en ce que** lors d'une étape a), des composés de formule (5)
NH₂-ARYL-X (5)
dans laquelle ARYL a la signification précédente et X = Cl, Br, I ou -OSO₂-R⁸ avec R⁸ = alkyle en C₁-C₁₀, perhalogénoalkyle en C₁-C₁₀, arylalkyle en C₇-C₁₅ ou aryle en C₆-C₂₄, ou R⁸ = alkyle en C₁-C₁₀ ou aryle en C₆-C₂₄ qui sont substitués une ou plusieurs fois, mais toutefois pas en totalité, par alcoxy en C₁-C₁₀, Cl, Br ou F,
sont mis en réaction avec des composés de formule (6)
R¹-Z (6)
dans laquelle R¹ = -COOR³ ou -SO₂-R⁴, R³ et R⁴ pouvant être identiques ou différents et étant choisis dans le groupe : alkyle en C₁-C₁₀, alcényle en C₂-C₁₀, perhalogénoalkyle en C₁-C₁₀, arylalkyle en C₇-C₁₅ ou aryle en C₆-C₂₄, ou R³ ou R⁴ = alkyle en C₁-C₁₀ ou aryle en C₆-C₂₄, qui sont substitués une ou plusieurs fois, mais toutefois pas en totalité, par alcoxy en C₁-C₁₀, Cl, Br ou F, ou R¹ = -SO₂-NH- (alkyle en C₁-C₁₀), -SO₂-NH-(arylalkyle en C₇-C₁₅), -SO₂-NH- (aryle en C₆-C₂₄) ou -SO₂-(NR⁵R⁶), R⁵ et R⁶ représentant alkyle en C₁-C₁₀, ou NR⁵R⁶ formant ensemble un cycle de 5 à 7 éléments, et
Z représentant fluor, chlore, brome, iode ou -O-CO₂-(alkyle en C₁-C₁₀), -O-CO₂-(aryle en C₆-C₂₄), -O-CO₂-(arylalkyle en C₇-C₁₅), -OSO₂-(alkyle en C₁-C₁₀), -OSO₂-(aryle en C₆-C₂₄), -OSO₂-NH-(arylalkyle en C₇-C₁₅) ou - OSO₂-(arylalkyle en C₇-C₁₅) éventuellement substitué ou non substitué, pour former des composés de formule (7)
W-ARYL-X (7)
dans laquelle ARYL et X ont la signification précédente et W = -NHR¹ ou -N(R¹)₂, R¹ ayant la signification indiquée précédemment, et
lors d'une étape b), les composés de formule (7) sont mis en réaction avec des composés de formule (3)
R²-MeY (3)
dans laquelle R² a la signification précédente et Y = ligand anionique et Me est un métal choisi dans le groupe constitué par Mg, Ca, Mn, Zn,
en présence d'au moins une source de fer, pour former des composés de formule (1), et les composés de formule (1)
sont mis en réaction lors d'une étape c) en présence d'acides ou de bases pour former des composés de formule (4).

12. Procédé selon une ou plusieurs des revendications 1 à 11, **caractérisé en ce que** le procédé selon l'étape b) est réalisé en présence d'un solvant organique.

13. Procédé selon la revendication 12, **caractérisé en ce que** le solvant organique est choisi dans le groupe constitué par les éthers, les amines, les amides ou les mélanges de ces solvants.

14. Procédé selon la revendication 11, **caractérisé en ce que** les composés de formule (6) sont le dicarbonate de di-tert.-butyle.
